# EUROPEAN PATENT SPECIFICATION

(11) **EP 3 345 562 B1**
(45) Date of publication and mention of the grant of the patent: **19.02.2020**
(21) Application number: 18150284.0
(22) Date of filing: 04.01.2018
(51) Int. Cl.: A61B 18/14, A61B 34/20

(54) **BALLOON BASKET CATHETER**
BALLONKORBKATHETER
CATHÉTER À BALLONNET DE PANIER

(30) Priority: 05.01.2017 US 201715398874
(43) Date of publication of application: 11.07.2018
(73) Proprietor: Biosense Webster (Israel) Ltd., Yokneam 2066717 (IL)
(72) Inventor: BASU, Shubhayu, Irwindale, CA 91706 (US); FUENTES-ORTEGA, Cesar, Irwindale, CA 91706 (US)
(74) Representative: Carpmaels & Ransford LLP

(56) References cited:
- US-A- 6 056 744
- US-A- 6 142 993
- US-A1- 2009 299 355
- US-A1- 2010 204 560
- US-A1- 2015 105 645
- US-A1- 2017 042 614
- US-B1- 6 258 087

## Description

### FIELD OF THE PRESENT DISCLOSURE

This invention relates to electrophysiologic (EP) catheters, in particular, EP catheters for mapping and/or ablation in the heart.

### BACKGROUND

Mapping of electrical potentials in the heart is now commonly performed, using cardiac catheters comprising electrophysiological sensors for mapping the electrical activity of the heart. Typically, time-varying electrical potentials in the endocardium are sensed and recorded as a function of position inside the heart, and then used to map a local electrogram or local activation time. Activation time differs from point to point in the endocardium due to the time required for conduction of electrical impulses through the heart muscle. The direction of this electrical conduction at any point in the heart is conventionally represented by an activation vector, which is normal to an isoelectric activation front, both of which may be derived from a map of activation time. The rate of propagation of the activation front through any point in the endocardium may be represented as a velocity vector. Mapping the activation front and conduction fields aids the physician in identifying and diagnosing abnormalities, such as ventricular and atrial tachycardia and ventricular and atrial fibrillation, that result from areas of impaired electrical propagation in the heart tissue.

Localized defects in the heart's conduction of activation signals may be identified by observing phenomena such as multiple activation fronts, abnormal concentrations of activation vectors, or changes in the velocity vector or deviation of the vector from normal values. Examples of such defects include re-entrant areas, which may be associated with signal patterns known as complex fractionated electrograms. Once a defect is located by such mapping, it may be ablated (if it is functioning abnormally) or otherwise treated so as to restore the normal function of the heart insofar as is possible. As an illustration, cardiac arrhythmias including atrial fibrillation, may occur when regions of cardiac tissue abnormally conduct electric signals to adjacent tissue, thereby disrupting the normal cardiac cycle and causing asynchronous rhythm. Procedures for treating arrhythmia include disrupting the origin of the signals causing the arrhythmia, as well as disrupting the conducting pathway for such signals, such as by forming lesions to isolate the aberrant portion. Thus, by selectively ablating cardiac tissue by application of energy via a catheter, it is sometimes possible to cease or modify the propagation of unwanted electrical signals from one portion of the heart to another. The ablation process destroys the unwanted electrical pathways by formation of non-conducting lesions.

A number of advantages may be obtained by providing a catheter having multiple electrodes to allow for mapping larger regions and/or for creating a plurality of lesions either simultaneously or without the need to reposition the catheter, such as by employing a basket-shaped electrode assembly. Examples are described in commonly assigned U.S. Pat. Nos. 5,772,590, 6,748,255 and 6,973,340. Basket catheters feature a plurality of spines, which are connected at their distal end as well as the proximal end. The spines may be deployed in an expanded arrangement wherein at least a portion of each spine extends radially outwardly from the catheter body or in a collapsed arrangement wherein each spine is disposed generally along the longitudinal axis of the catheter body. The collapsed arrangement facilitates advancing the electrode assembly to the desired location in the patient's body, such as through the vasculature in a percutaneous approach. When the electrode assembly assumes the expanded arrangement, one or more of the electrodes on the spines are brought into contact with tissue to allow for measurement of electrical signals and/or ablation of tissue.

By employing multiple spines, these electrode assemblies are adapted to provide an array of electrodes to occupy a three dimensional space defined by the anatomy of the patient, such as a chamber of the heart or an ostium vessel for example. Generally, it is desirable for the spines to be distributed evenly when expanded to provide uniform coverage of the electrode array over the space in which the spines are deployed. Alternatively, it may be desirable to distribute the spines in an uneven, but defined configuration in order to concentrate the electrodes at one or more regions of the space. However, conventional multiple spine electrode assemblies may not deploy with the spines in the intended configuration. For example, in a basket-shaped electrode assembly, they are secured only at their proximal and distal ends and the spines may not assume their intended radial distribution, particularly at locations that are farther away from the secured ends. Notably, the spines may bunch together more closely or may splay apart to a greater degree than desired. The tendency of the multiple spine electrode assemblies to assume such suboptimal distributions may be exacerbated by irregularities in a patient's anatomy or by friction with tissue.

US Patent 6, 142,993 discloses a collapsible electrode catheter assembly having a delivery system for controlling a catheter guide tube having a catheter distal end assembly thereon. The catheter distal end assembly has an electrode structure on an expanding assembly for enlarging the electrode structure in an expanded condition.

Published US Patent Application US 2010/0204560 A1 relates to a tissue electrode assembly including a membrane configured to form an expandable, conformable body that is deployable in a patient. The assembly further includes a flexible circuit positioned on a surface of the membrane and comprising at least one base substrate layer, at least one insulating layer and at least one planar conducting layer.

US Patent 6,056,744 discloses a sphincter treatment apparatus including an energy delivery device introduction member including a plurality of arms. Each arm has distal and proximal sections. The distal sections of the arms are coupled as are the proximal sections of the arms. The energy delivery device introduction member is configured to be introduced in the sphincter in a non-deployed state, expand to a deployed state to at least partially dilate the sphincter.

Accordingly, there is a need for a basket-shaped electrode assembly that helps maintain a desired relationship between the spines when deployed in their expanded arrangement. Similarly, there is a need for a basket-shaped electrode assembly in which the spines are stabilized with respect to each other. It would also be desirable to provide basket-shaped electrode assembly designs that readily take on different expanded arrangements to more readily contact areas or features of a patient's anatomy. The techniques of this disclosure as described in the following materials satisfy these and other needs.

### SUMMARY

The present disclosure is directed to a catheter with an elongated catheter body having a proximal end, a distal end and a lumen and a electrode assembly mounted at the distal end of the catheter body. The electrode assembly has at least two spines and an inflatable balloon in fluid communication with the elongated catheter body lumen. Each spine has at least one electrode and a proximal end attached at the distal end of the catheter body. Each spine has a collapsed arrangement in which the spines are arranged generally along a longitudinal axis of the catheter body and an expanded arrangement in which at least a portion of each spine bows radially outwards from the longitudinal axis. The inflatable balloon is disposed within the spines and the electrode assembly has a non-projecting distal portion when in the expanded arrangement. The non-projecting distal portion of the electrode assembly is formed by a portion of the balloon having a distal end being inverted within the balloon when in the expanded arrangement.

In one aspect, the inflatable balloon may be secured to each spine at a location distal of the proximal end of the spine. Each spine may have a length and the inflatable balloon may be secured to each spine along a portion of the spine length. Further, the inflatable balloon may be secured to each spine along the spine length.

In one aspect, the inflatable balloon may be configured to maintain a desired radial distribution of the spines relative to each other when in the expanded arrangement.

In one aspect, the inflatable balloon may be formed from a compliant material, semi-compliant material or a non-compliant material.

In one aspect, the hybrid electrode assembly may have an expander with proximal and distal ends, the expander slidably disposed within the lumen, such that the hybrid electrode assembly has the expanded arrangement when the expander is moved to a longitudinally proximal position relative to the catheter body and has the collapsed arrangement when the expander is moved to a longitudinally distal position relative to the catheter body. The hybrid electrode assembly may be configured to assume different conformations in the expanded arrangement based at least in part on a relative longitudinal position of the expander relative to the catheter body. The expander may be secured to a distal end of the balloon.

In one aspect, each spine may have a preshaped configuration corresponding to the expanded arrangement. Each spine may be formed from a shape memory material. In some embodiments, each spine may have a flexible core with a non-conductive covering. In some embodiments, each spine may include a flex circuit.

This disclosure also include a method for treatment involving providing a catheter with an elongated catheter body having a proximal end, a distal end, a lumen and a hybrid electrode assembly mounted at the distal end of the catheter having at least two spines and an inflatable balloon in fluid communication with the elongated catheter body lumen, each spine having at least one electrode and proximal end attached at the distal end of the catheter body. The distal end of the catheter with the hybrid electrode assembly may be advanced to a desired region within a patient in a collapsed arrangement with the spines arranged generally along a longitudinal axis of the catheter body and the hybrid electrode assembly may be caused to assume an expanded arrangement having a non-projecting distal portion in which the spines are positioned radially outwards from the longitudinal axis of the catheter body so that at least one electrode is in contact with tissue.

In one aspect, the hybrid electrode assembly assumes the expanded arrangement at least in part due to a preshaped configuration of the spines.

In one aspect, causing the hybrid electrode assembly to assume the expanded arrangement involves delivering inflation fluid to the inflatable balloon through the lumen of the catheter body. Adjusting the amount of delivered inflation fluid may alter the conformation of the hybrid electrode assembly in the expanded arrangement at the desired region within the patient

In one aspect, the hybrid electrode assembly may also have an expander with proximal and distal ends, the expander slidably disposed within the catheter body lumen, wherein distal ends of the spines are attached to the expander. The hybrid electrode assembly may be caused to assume the expanded arrangement by moving the expander to a longitudinally proximal position relative to the catheter body. Adjusting the longitudinal position relative to the catheter body may alter the conformation of the hybrid electrode assembly in the expanded arrangement at the desired region within the patient.

In one aspect, causing the hybrid electrode assembly to assume the expanded arrangement may create a region of the hybrid electrode assembly with a flattened disc-shaped configuration. The flattened disc-shaped configuration may include the non-projecting distal end of the hybrid electrode assembly

In one aspect, the method may involve receiving electrical signals from the at least one electrode in contact with tissue. The method may further involve moving the hybrid electrode assembly to a new position within the patient while maintaining the expanded arrangement and receiving electrical signals from the at least one electrode in the new position.

In one aspect, the method may involve altering the conformation of the hybrid electrode assembly in the expanded arrangement at the desired region within the patient.

### BRIEF DESCRIPTION OF THE DRAWINGS

Further features and advantages will become apparent from the following and more particular description of the preferred embodiments of the disclosure, as illustrated in the accompanying drawings, and in which like referenced characters generally refer to the same parts or elements throughout the views, and in which:
FIG. 1 is a top plan view of a catheter of the present invention, with a hybrid electrode assembly, according to one embodiment.
FIG. 2 is an end view of the hybrid electrode assembly shown in FIG. 1.
FIG. 3 is a schematic view of a spine having a flexible core with a non-conductive covering in a preshaped expanded arrangement, according to one embodiment.
FIG. 4. is a schematic view of flex circuit spine construction, according to one embodiment.
FIG. 5 is a schematic cross sectional view of the flex circuit spine construction of FIG. 4, according to one embodiment.
FIG. 6 is a schematic view of cage formed from spines prior to mounting an inflatable balloon, according to one embodiment.
FIG. 7 is a schematic view of a hybrid electrode assembly positioned within the left atrium, according to one embodiment.
FIG. 8 is a schematic view of a roving operation that may be performed with a hybrid electrode assembly, according to one embodiment.
FIGs. 9-11 are schematic illustrations of variable conformations of a hybrid electrode assembly to match pulmonary veins of different shapes and sizes, according to one embodiment.
FIGs. 12-14 are schematic illustrations of variable conformations of a hybrid electrode assembly that may be used for a stamping operation, according to one embodiment.
FIGs. 15-17 are schematic illustrations of variable conformations of a hybrid electrode assembly corresponding to different relative longitudinal positions of an expander, according to one embodiment.
FIG. 18 is a schematic illustration of an invasive medical procedure using a hybrid electrode assembly, according to one embodiment.
FIG. 19 is an elevational view of a hybrid electrode assembly with flex circuit spines, according to one embodiment.
FIG. 20 is an end view of the hybrid electrode assembly shown in FIG. 19.
FIG. 21 is a schematic illustration of a grooved balloon with a hemispherical distal region and a conical proximal region, according to one embodiment.
FIG. 22 is an elevational view of flex circuit spines disposed within a grooved balloon of a hybrid electrode assembly, according to one embodiment.
FIG. 23 is an end view of the hybrid electrode assembly shown in FIG. 22.

### DETAILED DESCRIPTION

At the outset, it is to be understood that this disclosure is not limited to particularly exemplified materials, architectures, routines, methods or structures as such may vary. Thus, although a number of such options, similar or equivalent to those described herein, can be used in the practice or embodiments of this disclosure, the preferred materials and methods are described herein.

It is also to be understood that the terminology used herein is for the purpose of describing particular embodiments of this disclosure only and is not intended to be limiting.

The detailed description set forth below in connection with the appended drawings is intended as a description of exemplary embodiments of the present disclosure and is not intended to represent the only exemplary embodiments in which the present disclosure can be practiced. The term "exemplary" used throughout this description means "serving as an example, instance, or illustration," and should not necessarily be construed as preferred or advantageous over other exemplary embodiments. The detailed description includes specific details for the purpose of providing a thorough understanding of the exemplary embodiments of the specification. It will be apparent to those skilled in the art that the exemplary embodiments of the specification may be practiced without these specific details. In some instances, well known structures and devices are shown in block diagram form in order to avoid obscuring the novelty of the exemplary embodiments presented herein.

For purposes of convenience and clarity only, directional terms, such as top, bottom, left, right, up, down, over, above, below, beneath, rear, back, and front, may be used with respect to the accompanying drawings. These and similar directional terms should not be construed to limit the scope of the disclosure in any manner.

Unless defined otherwise, all technical and scientific terms used herein have the same meaning as commonly understood by one having ordinary skill in the art to which the disclosure pertains.

Finally, as used in this specification and the appended claims, the singular forms "a, "an" and "the" include plural referents unless the content clearly dictates otherwise.

This disclosure is directed to a catheter having a hybrid electrode assembly. As noted above, certain types of electrical activity within a heart chamber are not cyclical. Examples include arterial flutter or arterial fibrillation, and ventricular tachycardia originating in scars in the wall of the ventricle that have resulted from infarcts. To analyze or 'map' this type of electrical activity, it is desirable to deploy a plurality of electrodes in contact with the tissue forming the chamber walls with the hybrid electrode assembly. In other embodiments, RF energy may be delivered to selected treatment areas for ablation based therapies, including for example, isolation of a source of irregular electrical signals by blocking electrical conduction. Focal ablations using unipolar devices benefit from targeted delivery of RF energy along with localized feedback of catheter placement, both spatially and with respect to tissue engagement. However, focal ablation procedures typically involve relative long procedure times as a result of the physician needing to stich a series of "quantized" RF ablation to form a lesion having the desired characteristics, such as the creation of a continuous circumferential block which surrounds the ostium of the targeted vein. Additionally, the use of a focal unipolar electrode requires substantial physician skill levels augmented with peripheral navigation systems in order to accurately and reliably position the electrodes. Correspondingly, use of the hybrid electrode assembly may present the opportunity to simultaneously record electrical signals and/or deliver ablation energy at a plurality of locations. The hybrid electrode assembly features multiple spines having an expanded arrangement with a desired distribution of the spines in relation to each other that may be maintained by an inflated balloon. The interactions between the spines and the balloon may also allow the hybrid electrode assembly to assume different conformations to more readily access desired areas of the patient's anatomy.

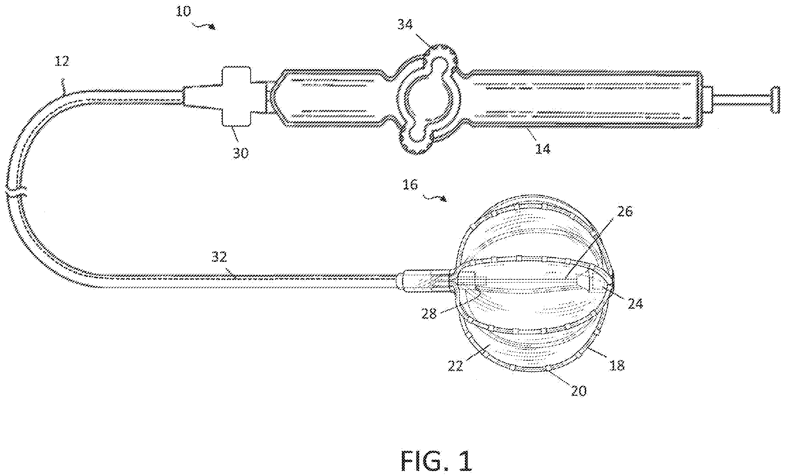

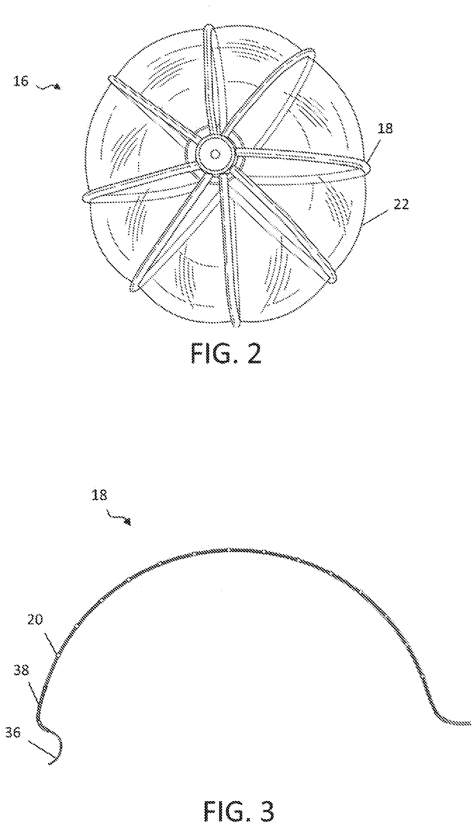

To help illustrate aspects of this disclosure, an exemplary embodiment of an electrophysiologic catheter with a multiple spine electrode assembly is shown schematically in FIG. 1. Catheter 10 comprises an elongated catheter body 12 having proximal and distal ends, a control handle 14 at the proximal end of the catheter body 12, and a hybrid electrode assembly 16 comprising a plurality of spines 18, with proximal ends of spines 18 mounted at the distal end of the catheter body 12. Each spine 18 may carry one or more electrodes 20 that may be located at any location and/or distribution along the length of spine 18. Spines 18 are shown in FIG. 1 in an expanded arrangement, with inflatable balloon 22 positioned within the space defined by spines 18. Balloon 22 may be secured to each spine 18 at least at an intermediate location between the proximal and distal ends of spine 18 to stabilize them with respect to each other. Depending upon the embodiment, each spine 18 may be secured to balloon 22 at multiple locations or substantially along their length to obtain a desired degree of stability and/or to assume different expanded arrangements as will be described in further detail below. An end view of the hybrid electrode assembly 16 shown in FIG. 1 is schematically depicted in FIG. 2. In this embodiment, spines 18 are distributed about the radius of balloon 22 substantially evenly. Alternatively, however, any desired distribution of spines 18 may be employed.

Balloon 22 may be formed from any suitable material, such as biocompatible polymers such as polyamides, polyesters, aramids, polyethylenes, polyurethanes and others, and may be compliant, semi-compliant, super compliant or non-compliant as warranted by the desired characteristics. As used herein, the term "compliant" means the balloon may be deformed, such as through expansion, when inflated and by forces transmitted via the spines and the term "relatively non-compliant" means the balloon does not substantially deform. Balloon 22 may be secured to spines 18 using any suitable technique, including without limitation adhesives, mechanical fasteners, heat bonding and the like. The number of attachments between spines 18 and balloon 22 and their locations, as well as the choice of balloon material, may be tailored to achieve any combination of desired characteristics. For example, attachment at intermediate locations along spines 18 may impart a relatively greater amount of stability in the radial positioning, helping to prevent migration of spines 18 towards or away from each other. Further, providing more points of attachment along the length of spines 18 may cause the spines to more closely assume an expanded arrangement dictated by the inflated shape of balloon 22. Conversely, reducing the number or areas of attachment between spines 18 and balloon 22 may allow hybrid electrode assembly to assume expanded arrangements other than the balloon shape. Still further, use of a relatively more compliant balloon material may allow the characteristics of spines 18, such as those formed from shape memory materials or preshaped configurations to have more influence on the expanded arrangement, while a less compliant balloon material may constrain spines 18 more closely to the balloon shape.

As described below, some embodiments of hybrid electrode assembly 16 may exhibit a manually adjustable degree of expansion to change its configuration and help conform to the tissue in the region in which it is deployed. As such, compliant balloon 22 may impart a stabilizing force to spines 18 over a range of relative distances between adjacent spines. For example, the distance between a pair of adjacent spines 18 may vary depending on the degree of expansion. So long as the distances are within the range of compliance of the balloon material, each spine 18 may be constrained by the resilience of balloon 22 so that it is stabilized with respect to its adjacent spines. Alternatively, balloon 22 may be formed from a relatively non-compliant material when it is desired that spines 18 maintain a single defined distance between each other when in the expanded arrangement. Notably, even for a non-compliant material, under inflation of the balloon may still allow for changes in the degree of expansion and correspondingly, changes in the conformation of the expanded arrangement to increase the ability of hybrid electrode assembly 16 to interact with the patient's anatomy. Accordingly, it will be appreciated that the conformation of hybrid electrode assembly 16 when in an expanded arrangement may be controlled at least in part by varying the inflation characteristics of balloon 22. For example, the pressure to which balloon 22 is inflated may be adjusted to impart different conformations. As another example, the volume of the inflation fluid introduced may also be adjusted to achieve control over the conformation of the expanded arrangement.

In the depicted embodiment, hybrid electrode assembly 16 comprises eight spines 18. Each spine 18 has a proximal end attached at the distal end of the catheter body 12 and a distal end secured to the distal ends of the other spines, either directly or indirectly. As an example of a suitable construction, each spine 18 may have a flexible core, such as an internal strut, a substrate, or any other structural member comprising a metal or plastic material having characteristics such as resiliency and/or shape memory that allow the spines 18 to assume their expanded and collapsed arrangements per the discussion below. Hybrid electrode assembly 16 may be a discrete element that is joined to catheter body 12 or may comprise one or more elements that extend from catheter body 12. Further, as will be described in more detail below, the polar region of hybrid electrode assembly 16 may have a non-protruding configuration when in an expanded arrangement so that electrodes 20 positioned in this region may contact tissue more readily. In some embodiments, the overall diameter of hybrid electrode assembly 16 may be in the range of approximately 1 mm to 4 mm, such as 2.5 mm, to allow adjustment of the position when deployed in an expanded arrangement, so that different regions may be interrogated and/or treated. In other embodiments, the overall diameter of hybrid electrode assembly 16 may be configured to substantially fill the volume in which is deployed, such as having a diameter of approximately 5 mm for ventricular applications.

As will be recognized by one skilled in the art, the number of spines 18 may vary as desired depending on the particular application, so that the catheter 10 has at least two spines, and may have three or more spines up to twelve or more. Spines 18 are moveable between an expanded arrangement, wherein, for example, each spine extends radially outwardly from the catheter body 12, and a collapsed arrangement, wherein, for example, each spine is disposed generally along a longitudinal axis of the catheter body 12 so that the spines are capable of fitting within a lumen of a guiding sheath, as discussed further below.

As noted, each spine 18 carries at least one electrode mounted along its length. In the depicted embodiment, electrodes 20 are mounted along the length of spines 18 on the non-conductive covering. As desired, electrodes 20 may be configured as unipolar, bipolar or both and may be diagnostic electrodes, ablation electrodes, reference electrodes or others. Hybrid electrode assembly 16 may also have at least one position sensor 24, such as at a distal end as shown, to help determine the orientation and/or position of hybrid electrode assembly 16 when deployed within a patient as described below.

The hybrid electrode assembly 16 may also include an expander 26 is generally coaxial with the catheter body 12 and extends from the proximal end of catheter body 12 through the central lumen and is attached, directly or indirectly, to the distal ends of spines 18. For example, the expander may be secured to a distal end of balloon 22. The expander 26 is afforded longitudinal movement relative to the catheter body so that it can move the distal ends of the spines 18 proximally or distally relative to the catheter body 12 to radially expand and contract, respectively, the electrode assembly. Since the proximal ends of spines 18 are secured to the catheter body 12, relative movement of expander 26 in the proximal direction shortens the distance between the distal and proximal ends of spines 18, causing them to bow outwards into an expanded arrangement. The expander 26 comprises a material sufficiently rigid to achieve this function. In an embodiment, the expander 26 comprises braided polyimide tubing having inner and outer layers of polyimide with a braided stainless steel mesh there between, as is generally known in the art. As will be appreciated, different relative amounts of movement of the expander 26 along the longitudinal axis may affect the degree of bowing, such as to enable the spines 18 to exert greater pressure on the atrial tissue for better contact between the tissue and the electrodes on the spines. Thus, a user can change the shape of the electrode assembly by adjusting the longitudinal extension or withdrawal of the expander.

Alternatively or in addition, spines 18 may include a material that facilitates assuming the expanded arrangement, so that spines 18 may have a preshaped configuration corresponding to the desired shape of hybrid electrode assembly 16 when deployed within a patient. For example, spines 18 may have a core formed from a shape memory material to help spines 18 assume the expanded and collapsed arrangements. Notably, nickel-titanium alloys known as nitinol may be used. At body temperature, nitinol wire is flexible and elastic and, like most metals, nitinol wires deform when subjected to minimal force and return to their shape in the absence of that force. Nitinol belongs to a class of materials called Shaped Memory Alloys (SMA) that have interesting mechanical properties beyond flexibility and elasticity, including shape memory and superelasticity which allow nitinol to have a "memorized shape" that is dependent on its temperature phases. The austenite phase is nitinol's stronger, higher-temperature phase, with a simple cubic crystalline structure. Superelastic behavior occurs in this phase (over a 50°-60°C temperature spread). Correspondingly, the martensite phase is a relatively weaker, lower-temperature phase with a twinned crystalline structure. When a nitinol material is in the martensite phase, it is relatively easily deformed and will remain deformed. However, when heated above its austenite transition temperature, the nitinol material will return to its pre-deformed shape, producing the "shape memory" effect. The temperature at which nitinol starts to transform to austenite upon heating is referred to as the "As" temperature. The temperature at which nitinol has finished transforming to austenite upon heating is referred to as the "Af' temperature.

Thus, depending on the embodiment, the transition between the collapsed arrangement and the expanded arrangement may be caused by the characteristics of spines 18, by mechanistic actuation or by a combination. For example, spines 18 may have a preshaped configuration when not restrained by a guiding sheath, causing it to expand radially outwards to assume the expanded configuration. Alternatively, a mechanism, such as expander 26, may be used to adjust the relative distance between the distal and proximal ends of spines 18, causing hybrid electrode assembly 16 to bow outwards into the expanded arrangement. Regardless of the manner in which expansion occurs, hybrid electrode assembly 16 may have one or more expanded arrangements in which spines 18 form generally spherical, ovoid, ellipsoidal, conical or other closed shape, but may also be designed to take on other shapes which may be regular or irregular as well as being open or closed. For example, embodiments discussed below may include conformations in which hybrid electrode assembly 16 exhibits a flattened, disc-shaped region at the non-projecting distal end.

Catheter body 12 comprises an elongated tubular construction having a single, axial or central lumen but can optionally have multiple lumens along all or part of its length if desired. For example, as indicated in the embodiment shown in FIG. 1, lumen 28 may be provided to convey inflation fluid to the interior of balloon 22 introduced through adapter 30 to control expansion of hybrid electrode assembly 16 and/or stabilize spines 18. Catheter body 12 is flexible, i.e., bendable, but substantially non-compressible along its length. Catheter body 12 can be of any suitable construction and made of any suitable material, such as by using an outer wall of polyurethane or PEBAX® (polyether block amide). The wall may have an imbedded braided mesh of stainless steel or the like, as is generally known in the art, to increase torsional stiffness of the catheter body 12 so that, when the control handle 14 is rotated, the distal end of the catheter body 12 will rotate in a corresponding manner.

The length of the catheter body 12 is not critical, but may range from about 90 cm to about 120 cm, such as about 110 cm. The outer diameter of the catheter body 12 is also not critical, but generally be adapted to present an outer diameter sufficient to accommodate the construction of hybrid electrode assembly 16 and any associated leads, irrigation lumens, puller wires, position or other sensors and the like while retaining an insertion profile that allows advancement through the patient's vasculature. In some embodiments, catheter body 12 may be about 10 french or less, such as 8 french or 7 french. Likewise, the thickness of the outer wall of catheter body 12 is not critical but may be thin enough to provide a lumen or lumens of sufficient size. An example of a catheter body construction suitable for use in connection with the present invention is described and depicted in U.S. Pat. No. 6,064,905.

In some embodiments, catheter shaft 12 may be deflectable to impart further control over which areas of tissue are contacted by asymmetric basket-shaped electrode assembly 16. At least one puller wire 32 may be secured at its distal end to a distal portion of catheter shaft 12 and at its proximal end to an actuator 34 on control handle 14. Rotating, or otherwise manipulating actuator 34 may place puller wire 32 under tension, producing a deflection of catheter shaft 12 away from its longitudinal axis. One puller wire may be employed to impart a uni-directional deflection, while an additional puller wire may provide bi-directional deflection. Examples of suitable construction details for deflectable catheters for are described in U.S. Patent No. 7,377, 906, entitled "Steering Mechanism For Bi-Directional Catheter," and U.S. Patent No. 8,137,308, entitled "Catheter With Adjustable Deflection Sensitivity,". Other suitable techniques may also be employed to provide deflection as desired.

According to the techniques of this disclosure, each spine 18 may be formed using any suitable construction. For example, FIG. 3 schematically illustrates one spine 18 having a flexible core 36 with a non-conductive covering 38. Flexible core 36 may be chosen to have sufficient resiliency to allow transition from the collapsed and expanded arrangements, and in some embodiments, may be preshaped as noted to help assume a desired expanded arrangement. In one embodiment, flexible core 36 may be a shape memory material as described above. In other embodiments, the flexible core 36 may be formed from a polymer or other non-conductive material which may preclude the need for a separate covering. One or more electrodes 20 are mounted over the non-conductive covering 38, which may be a suitable biocompatible plastic tubing, such as polyurethane, polyether or polyimide materials. For example, electrodes 20 may be configured as ring electrodes that are swaged over non-conductive covering 38. Since interior areas of such ring electrodes are blocked by the balloon, the may be less susceptible to far field signals and provide more accurate measurements.

Alternatively, spines 18 may also be formed in other suitable manners, including through use of a structural substrate 40 with a polymeric layer 42 as indicated in the partial detail view depicted in FIG. 3. Such embodiments may employ construction techniques used to create flexible circuits, or "flex circuits," as known in the art. Polymeric layer 42 may be any suitable flexible polymer, such as polyester, polyimide, polyethylene napthalate (PEN), polyetherimide (PEI), fluropolymers (FEP), polyether ether ketone (PEEK) or the like, including copolymers. Generally, polymeric layer 42 may be provided with conductive elements, such as electrodes 20, as well as leads, traces and the like, as desired using metallic foil and photolithography or equivalent techniques, although suitable patterns of conductive tape may be laminated between layers of polymer or electrodeposition methods may also be used. For example, polymeric layer 42 may be coated with metallization layers to provide an interface. Suitable metals include gold, titanium, copper and the like, and may be deposited by sputtering or plating.

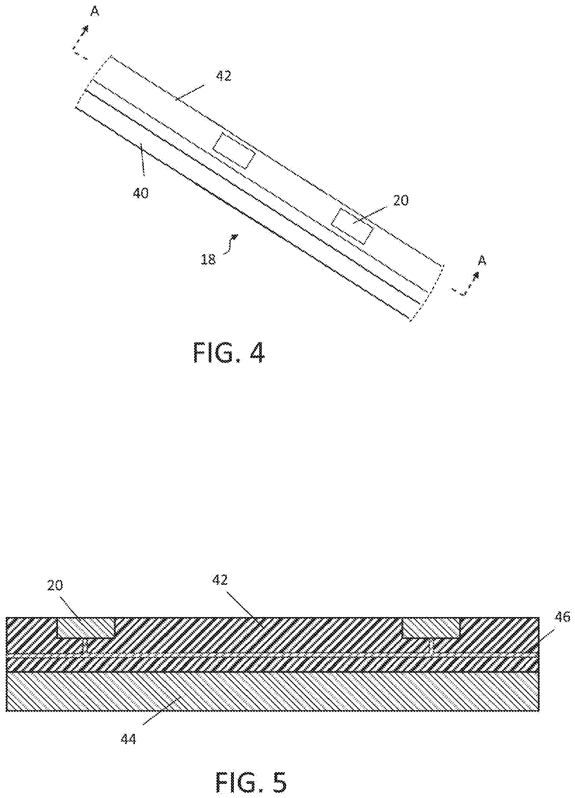

As depicted in FIG. 5, which is a cross sectional view taken at line A-A in FIG. 4, polymeric layer 34 is applied over substrate 40 and electrodes 20 are printed on top. This depiction is intended only to schematically illustrate the relationship between the various layers and is not to scale. For example, polymeric layer 42 may range from approximately 40 to 60 µm in some embodiments, but the thickness of this layer and the others may be adapted to provide the desired mechanical and electrical characteristics. Depending on the embodiment, electrodes 20 may be relatively flush with the surface of polymeric layer 42 or may protrude to facilitate tissue engagement. One or more conductive layers 44 may provide connectivity for electrodes 20. If different electrode configurations are employed, any combination and number of different configurations may be provided as warranted by the intended application. By employing the flex circuit techniques, any number of electrodes 20 may be readily positioned at any location on spines 18. For example, electrodes 20 may be provided in a density of approximately 0.25 electrodes/cm² or greater to allow for mapping of an area in which the catheter is deployed with improved resolution. As will be appreciated, the construction techniques used in forming a flex circuit allow for significant design freedom with regard to the number and positioning of electrodes 20. Suitable electrode materials include gold, titanium, iridium and platinum, as well as alloys and oxides of these metals.

Accordingly, as with other spine constructions, a hybrid electrode assembly 16 having spines 18 with flex circuits may have a three-dimensional shape that can be easily collapsed to be fed into a guiding sheath and then readily returned to its expanded arrangement upon delivery to the desired region of the patient upon removal of the guiding sheath. In some embodiments, substrate 40 may be formed from a nitinol hypotube by laser cutting or other similar techniques, to provide a monolithic framework. Depending on the embodiment, a 3mm tube having a wall thickness of approximately 8 to 9 mil may be used to form substrate 40. As an example of an alternative construction, spines 18 may be formed with polymeric layer 42 alone without a separate substrate 40. One exemplary material for such embodiments may be PEEK, such that spines 18 may be cut from a tube of PEEK or by using any other suitable technique. Further, balloon 22 may be configured to impart the desired conformation when in the expanded arrangement, reducing the need for spines 18 to contribute to the structural characteristics of the expanded arrangement. Independently, spines 18 may also help return hybrid electrode assembly 16 to the collapsed arrangement, such as by facilitating balloon 22 to fold along pleat lines formed by spines 18.

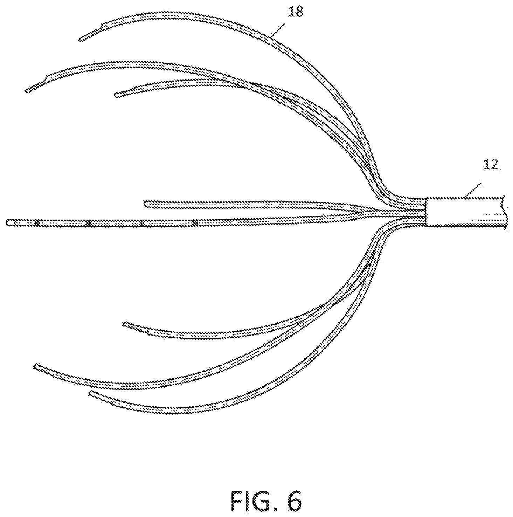

During construction, a cage formed by spines 18 may be formed initially as schematically shown in FIG. 6, so that balloon 22 (not shown in this view) may be positioned within the framework and suitably attached to spines 18, such as through adhesives, heat-bonding or any other method. As noted above, the distal ends of spines 18 may be secured together to form a structure similar to other basket-shaped electrode assemblies, or may form a spine structure that covers only on desired portions of the surface of balloon 22. Since spines 18 may have a preshaped configuration as discussed above, spines 18 may be used to dictate the overall shape of hybrid electrode assembly 16 with balloon 22 serving as a supporting structure, balloon 22 may provide the shape while spines 18 act as supporting structures, or a combination of these factors may be used to achieve the desired conformation of hybrid electrode assembly 16 when expanded.

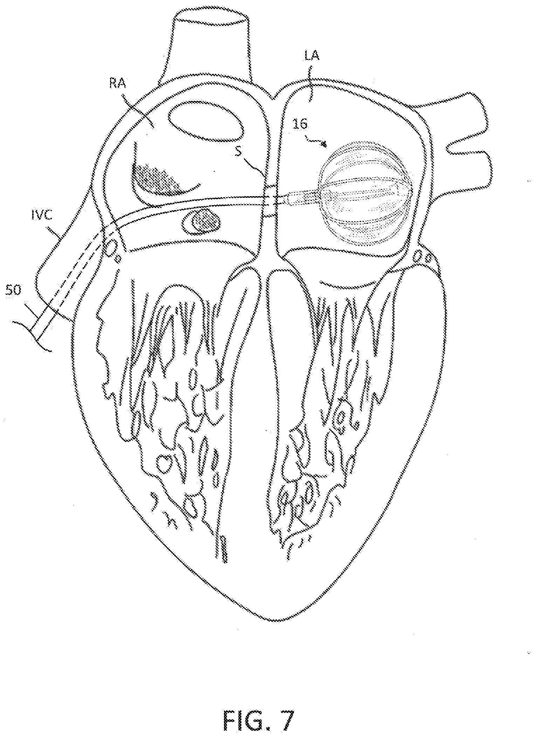

In one aspect, an electrophysiologist may introduce a guiding sheath, guidewire and dilator into the patient, as is generally known in the art. As an example, a guiding sheath for use in connection with the inventive catheter is an appropriately-sized PREFACE™ Braided Guiding Sheath (commercially available from Biosense Webster, Inc., Diamond Bar, CA). The guidewire is inserted, the dilator is removed, and the catheter is introduced through the guiding sheath whereby the guidewire lumen in the expander permits the catheter to pass over the guidewire. In one exemplary procedure as depicted in FIG. 7, the catheter is first introduced to the patient's heart (H) through the right atrium (RA) via the inferior vena cava (IVC), where it passes through the septum (S) in order to reach the left atrium (LA).

As will be appreciated, hybrid electrode assembly 16 may be deflected into its collapsed arrangement and constrained within guiding sheath 50 to allow catheter 10 to be passed through the patient's vasculature to the desired location. Once the distal end of the catheter reaches the desired location, e.g., the left atrium, guiding sheath 50 is withdrawn to expose the hybrid electrode assembly 16, allowing it to assume the expanded arrangement, with balloon 22 stabilizing spines 18 in an intended configuration. Alternatively, the hybrid electrode assembly 16 may be pushed through the guiding sheath 50 to expose the hybrid electrode assembly 16, allowing it to assume the expanded arrangement, with balloon 22 stabilizing spines 18 in an intended configuration.

As noted above, hybrid electrode assembly 16 may assume the expanded arrangement due to characteristics of spines 18, such as having a pre-shaped conformation, due to manipulation of expander 26 to adjust the relative position of the distal ends of spines 18 with respect the proximal ends, due to the degree to which balloon 22 has been inflated, or any combination of these factors.

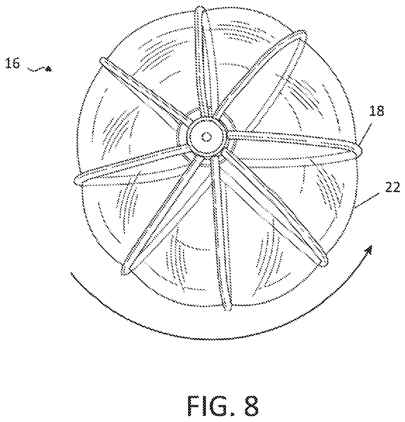

During use, one aspect associated with the embodiments of this disclosure is the capability to rapidly collect data points for an area of tissue being investigated. For example, after hybrid electrode assembly 16 has assumed an expanded arrangement, data may be collected corresponding to the current position of each electrode 20. To perform a more thorough mapping, it may be desirable to move hybrid electrode assembly 16 to successive new positions and/or orientations so that electrodes 20 may obtain readings from new locations. Techniques involving such movement may be termed "roving." One suitable roving operation may involve rotating hybrid electrode assembly 16 axially, schematically indicated by FIG. 8, such as by suitable manipulation of control handle 14. Further, the position of hybrid electrode assembly 16 may be adjusted as warranted by deflection of catheter shaft 12, such as by suitable manipulation of actuator 34.

As desired, hybrid electrode assembly 16 may remain in an expanded arrangement during the roving operation. Since balloon 22 has been inflated, it creates a surface across the radial space between spines 18. Accordingly, hybrid electrode assembly 16 exhibits a relatively smooth surface, reducing the abrupt transitions that would be associated with spines 18 alone. As noted above, the tension imparted between spines 18 by balloon 22 resists the tendency for the spines 18 to be deflected by friction with tissue when hybrid electrode assembly 16 is rotated. Consequently, the accuracy associated with mapping or other operations involving electrodes 20 may be increased. Further, the smooth transition provided by balloon 22 may reduce trauma that may be experienced by the tissue when hybrid electrode assembly 16 is rotated. By reducing drawbacks such as spine deflection or injury, balloon 22 may remain inflated and hybrid electrode assembly 16 in an expanded arrangement during roving to decrease the time associated to perform the operation, reducing or eliminating the need to transition between expanded and collapsed arrangements when reorienting hybrid electrode assembly 16.

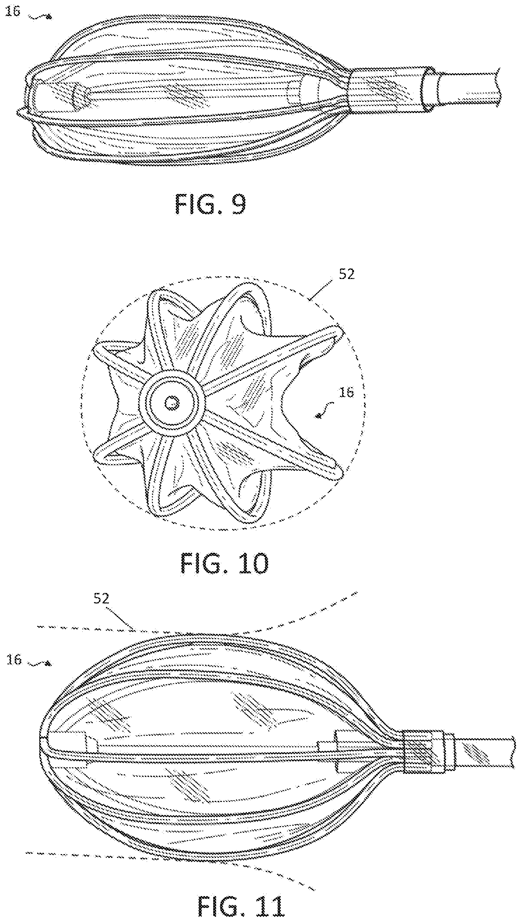

One aspect of hybrid electrode assembly 16 noted above is the ability to alter conformation when in an expanded arrangement by adjusting inflation of balloon 22 and/or relative longitudinal movement of expander 26. By altering the conformation of hybrid electrode assembly 16, it may be possible to accommodate a wider range of variability in the patient's anatomy, such as different shapes or sizes of pulmonary veins. For example, FIGs. 9-11 schematically depict three conformations of hybrid electrode assembly 16 that may be achieved according to the techniques of this disclosure. It should be appreciated that these are examples only and other degrees of inflation and/or expander 26 movement may achieve other shapes as desired to more closely match the region being investigated. In the context of this example, FIG. 9 illustrates hybrid electrode assembly 16 with balloon 22 in a relatively less inflated condition. The end view shown in FIG. 10 illustrates this conformation disposed within a pulmonary vein ostium 52. By tailoring the degree of inflation, the conformation of hybrid electrode assembly 16 may be adjusted to more closely fit the dimensions desired. Turning to FIG. 11, balloon 22 of hybrid electrode assembly 16 is shown in side view in a relatively more inflated condition, also positioned within pulmonary vein ostium 52.

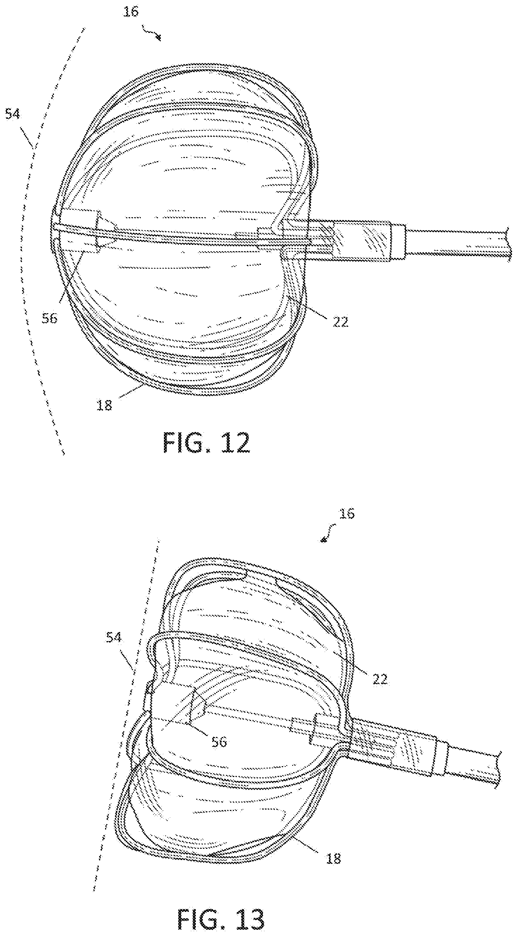

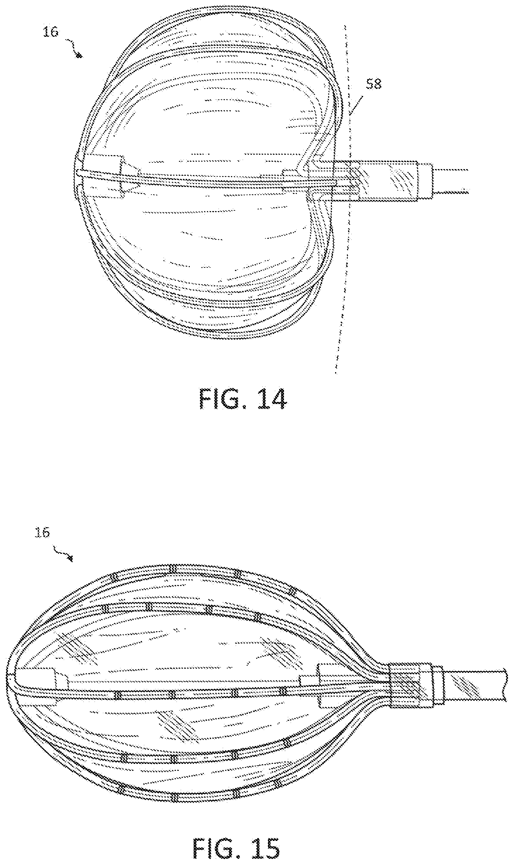

Further examples of the variable conformations of hybrid electrode assembly 16 are schematically depicted in FIGs. 12-14. For example, FIG. 12 illustrates hybrid electrode assembly 16 in a conformation as may be achieved by suitable adjustment in the degree of inflation of balloon 22 and/or manipulation of expander 26. Accordingly, the distal end of hybrid electrode assembly 16 may have a disc-shaped surface that is still convex, but is flattened with respect to other regions of hybrid electrode assembly 16 to increase the degree of contact with tissue 54 having a concave surface. Notably, the distal ends of spines 18 may be secured to hub 56, which may be positioned within the interior of balloon 22 when in the expanded arrangement. As a result, the distal end of hybrid electrode assembly 16 is non-protruding, allowing any electrodes located in the polar region to be brought into contact with tissue more readily. In comparison, conventional electrode assemblies have projecting distal ends that may prevent adjacent electrodes from contacting tissue. Further, the conformation shown in FIG. 13, which also may be achieved by suitable adjustment in the degree of inflation of balloon 22 and/or manipulation of expander 26, also having disc-shaped surface that is relatively flat to correspondingly increase the degree of contact with a flat surface of tissue 54. Further, adjustment in the degree of inflation of balloon 22 and/or manipulation of expander 26 may be used to impart a flattened surface at the proximal end of hybrid electrode assembly 16 as shown in FIG. 14. In this conformation, hybrid electrode assembly 16 may more readily contact other tissue surfaces, such as septal wall 58. Each of these conformations, as well as others that may be imparted to hybrid electrode assembly 16, may be used in a technique termed "stamping." During a stamping operation, the disc-shaped surface may be pressed against an area of tissue to record signals from electrodes 20 and then successively shifted to new positions to increase the density of measurements for a given area. The ability to successfully employ hybrid electrode assembly 16 in such stamping operations is improved by the non-projecting distal end as described above.

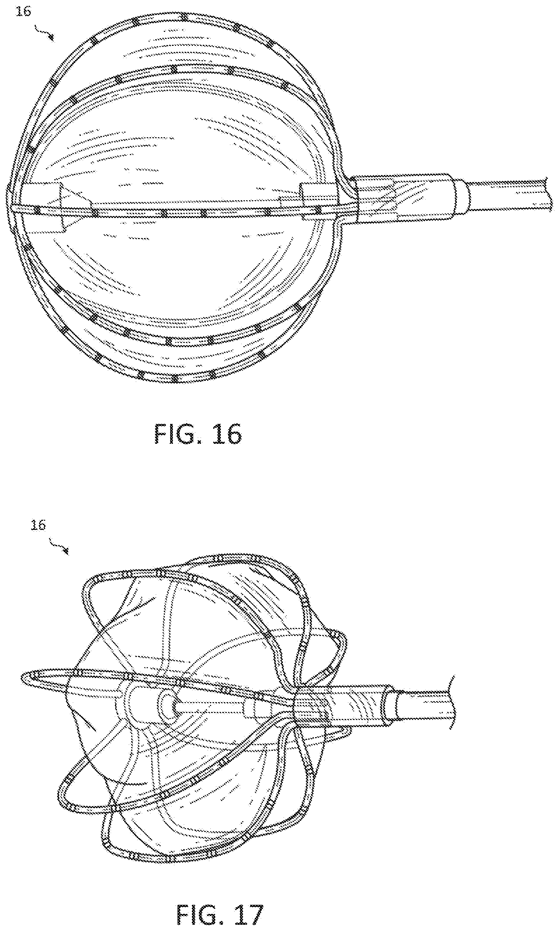

Still further examples of hybrid electrode assembly 16 conformations are schematically depicted in FIGs. 15-17. These examples help exhibit the effect of the relative longitudinal position of expander 26. In FIG. 15, a conformation of hybrid electrode assembly 16 is shown, which corresponds to a relatively more distal position of expander 26, increasing the distance between the proximal and distal ends of spines 18. Next, FIG. 16 depicts a conformation of hybrid electrode assembly 16 corresponding to an intermediate position of expander 26. Correspondingly, a conformation corresponding to expander 26 being at a relatively proximal position is shown in FIG. 17, with the distance between the proximal and distal ends of spines 18 having been reduced. Notably, spines 18 may be secured to balloon 22 at intermediate locations rather than along their length, allowing spines 18 to form a more angular configuration associated with conformations of hybrid electrode assembly 16 as shown in FIGs. 12-14.

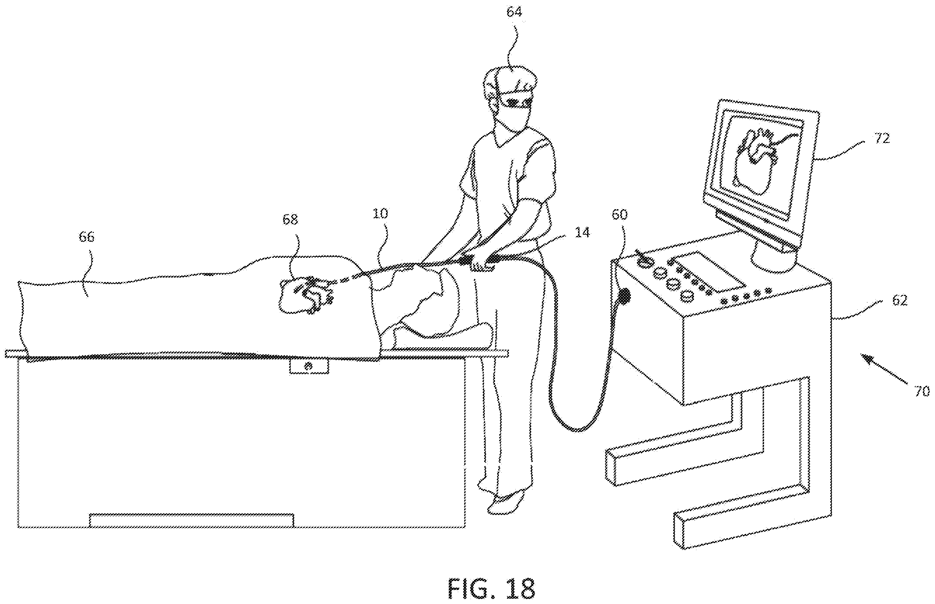

As will be appreciated, a procedure employing a hybrid electrode assembly with the techniques of this disclosure allow any desired operation involving measuring electrical signals and/or ablating tissue within a patient. To help illustrate use of the hybrid electrode assembly 16, FIG. 18 is a schematic depiction of an invasive medical procedure, according to an embodiment of the present invention. Catheter 10, with the hybrid electrode assembly 16 (not shown in this view) at the distal end may have a connector 60 at the proximal end for coupling the leads of the electrodes and sensors (not shown in this view) to a console 62 for recording and analyzing the signals they detect as well as for supplying ablating energy. An electrophysiologist 64 may insert the catheter 10 into a patient 66 in order to acquire electropotential signals from the heart 68 of the patient. The electrophysiologist 64 uses the control handle 14 attached to the catheter in order to perform the insertion. Console 62 may include a processing unit 70 which analyzes the received signals, and which may present results of the analysis on a display 72 attached to the console. The results are typically in the form of a map, numerical displays, and/or graphs derived from the signals. Processing unit 70 may also control the delivery of energy to the electrodes for creating one or more lesions, such as at locations associated with abnormal electrical activity identified by analyzing received signals.

Further, the processing unit 70 may also receive signals from position sensors, such as sensor 24 (not shown in this view). As noted, the sensor(s) may each comprise a magnetic-field-responsive coil or a plurality of such coils. Using a plurality of coils enables six-dimensional position and orientation coordinates to be determined. The sensors may therefore generate electrical position signals in response to the magnetic fields from external coils, thereby enabling processor 70 to determine the position, (e.g., the location and orientation) of the distal end of catheter 10 within the heart cavity. The electrophysiologist may then view the position of the hybrid electrode assembly 16 on an image the patient's heart on the display 72. By way of example, this method of position sensing may be implemented using the CARTO™ system, produced by Biosense Webster Inc. (Diamond Bar, Calif.) and is described in detail in U.S. Pat. Nos. 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1. As will be appreciated, other location sensing techniques may also be employed. If desired, at least two location sensors may be positioned proximally and distally with respect to hybrid electrode assembly 16. The coordinates of the distal sensor relative to the proximal sensor may be determined and, with other known information pertaining to the configuration of hybrid electrode assembly 16, used to find the positions of each of the electrodes 20.

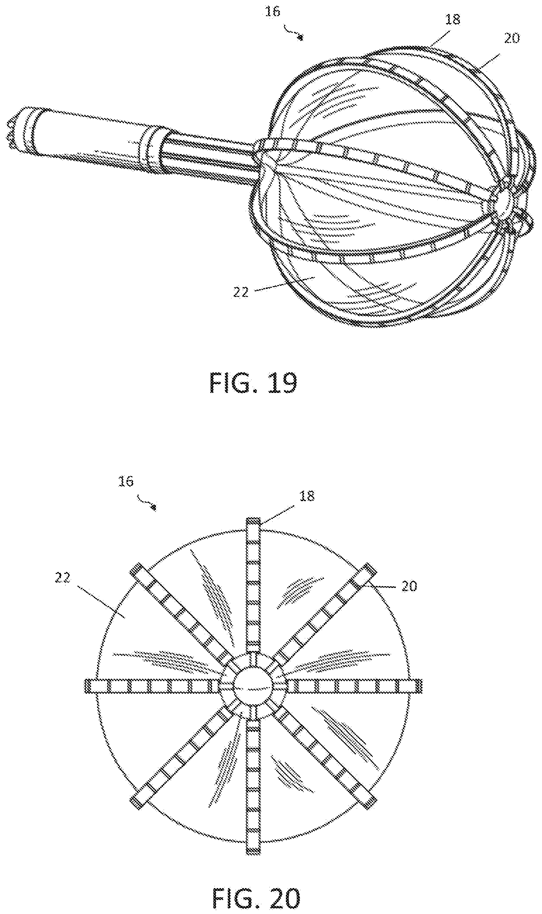

Another embodiment of hybrid electrode assembly 16 is schematically depicted in the elevational view of FIG. 19 and the end view of FIG. 20. As shown, spines 18 are formed from flex circuits and may be secured to balloon 22 in any suitable manner. Similar to the embodiments described above, the distal end of hybrid electrode assembly 16 is non-projecting, having a relatively smooth outer profile and a radius of curvature consistent with the portions adjacent the polar region. Accordingly, electrodes 20, including those positioned in the polar region, may be brought into contact with tissue more readily. Notably, the size of electrodes 20 is not necessarily shown to scale and accordingly may have any suitable surface area as warranted by the intended application.

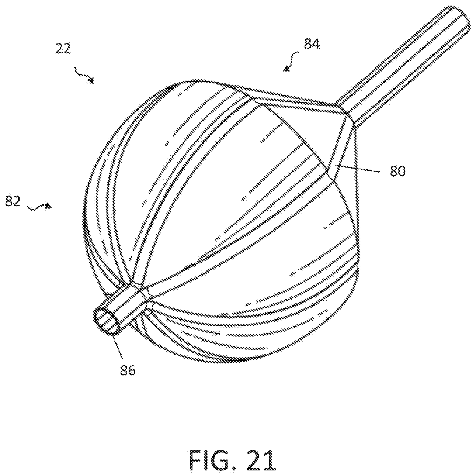

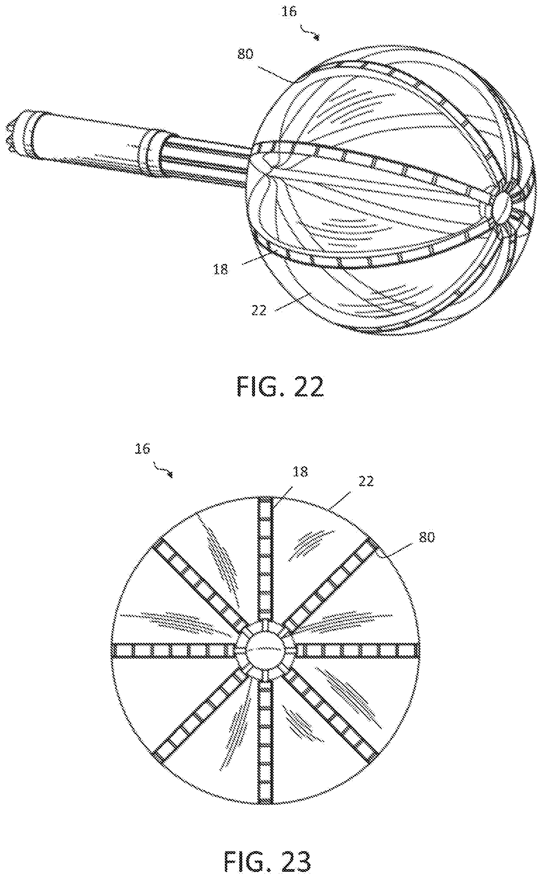

In a further aspect of this disclosure, balloon 22 may be formed with longitudinal grooves 80 to receive spines 18 as shown in the embodiment of balloon 22 schematically depicted in FIG. 21. Here, balloon 22 has a distal region 82 having a hemispherical configuration and a proximal region 84 with a conical configuration. As known in the art, balloon 22 may be formed from a tubular parison, such as being blow molded. Following formation of the balloon, a distal portion 84 may retain the tubular configuration of the parison. In order to achieve the non-projecting conformation discussed above, distal portion 82 may be inverted and drawn within the expanded profile of balloon 22. Correspondingly, balloon 22 will then have the non-projecting conformation and the distal portion 82 may be used to secure other elements, such as hub 56 (shown in FIGs. 12-14) or expander 26 (shown in FIG. 1).To help illustrate the effect of a grooved balloon, an embodiment of hybrid electrode assembly 16 is schematically depicted in an elevational view in FIG. 22 and an end view in FIG. 23. As shown, at least a portion of the cross-sectional profile of spines 18 may be disposed within grooves 80 of balloon 22. Correspondingly, hybrid electrode assembly 16 may exhibit a relatively smoother outer profile to facilitate movement across tissue, such as during repositioning to interrogate different tissue regions.

The preceding description has been presented with reference to presently disclosed embodiments of the invention. Workers skilled in the art and technology to which this invention pertains will appreciate that alterations and changes in the described structure may be practiced without meaningfully departing from the principle and scope of this invention. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. A catheter comprising:
an elongated catheter body (12) having a proximal end, a distal end and a lumen; and
an electrode assembly (16) mounted at the distal end of the catheter body and comprising at least two spines (18) and an inflatable balloon (22) in fluid communication with the elongated catheter body lumen, each spine having at least one electrode (20) and a proximal end attached at the distal end of the catheter body and wherein each spine has a collapsed arrangement in which the spines are arranged generally along a longitudinal axis of the catheter body and an expanded arrangement in which at least a portion of each spine bows radially outwards from the longitudinal axis, such that the inflatable balloon is disposed within the spines and the electrode assembly has a non-projecting distal portion when in the expanded arrangement, wherein the non-projecting distal portion of the electrode assembly is formed by a portion of the balloon having a distal end being inverted within the balloon when in the expanded arrangement.

2. The catheter of claim 1, wherein the inflatable balloon (22) is secured to each spine (18) at a location distal of the proximal end of the spine.

3. The catheter of claim 2, wherein each spine (18) has a length and wherein the inflatable balloon (22) is secured to each spine along a portion of the spine length.

4. The catheter of claim 3, wherein the inflatable balloon (22) is secured to each spine (18) along the spine length.

5. The catheter of claim 1, wherein the inflatable balloon (22) is configured to maintain a desired radial distribution of the spines (18) relative to each other when in the expanded arrangement.

6. The catheter of claim 1, wherein the inflatable balloon (22) comprises a compliant material, a non-compliant material, or a semi-compliant material.

7. The catheter of claim 1, further comprising an expander (26) having proximal and distal ends, the expander slidably disposed within the lumen, such that the electrode assembly (16) has the expanded arrangement when the expander is moved to a longitudinally proximal position relative to the catheter body (12) and has the collapsed arrangement when the expander is moved to a longitudinally distal position relative to the catheter body.

8. The catheter of claim 7, wherein the electrode assembly (16) is configured to assume different conformations in the expanded arrangement based at least in part on a relative longitudinal position of the expander (26) relative to the catheter body (12).

9. The catheter of claim 7, wherein the expander (26) is secured to a distal end of the balloon (22).

10. The catheter of claim 1, wherein each spine (18) has a pre-shaped configuration corresponding to the expanded arrangement.

11. The catheter of claim 10, wherein each spine (18) comprises a shape memory material.

12. The catheter of claim 1, wherein each spine (18) comprises a flexible core with a non-conductive covering.

13. The catheter of claim 1, wherein each spine (18) comprises a flex circuit.

## Patentansprüche

1. Katheter, umfassend:
einen länglichen Katheterkörper (12) mit einem proximalen Ende, einem distalen Ende und einem Lumen, und
eine Elektrodenanordnung (16), die an dem distalen Ende des Katheterkörpers montiert ist und mindestens zwei Grate (18) und einen inflatierbaren Ballon (22) in Fluidverbindung mit dem länglichen Katheterkörperlumen umfasst, wobei jeder Grat mindestens eine Elektrode (20) und ein an dem distalen Ende des Katheterkörpers angebrachtes proximales Ende hat und wobei jeder Grat eine zusammengeklappte Anordnung, in der die Grate allgemein entlang einer Längsachse des Katheterkörpers angeordnet sind, und eine expandierte Anordnung hat, in der sich mindestens ein Abschnitt jedes Grats von der Längsachse radial nach außen biegt, so dass der inflatierbare Ballon innerhalb der Grate angeordnet ist, und die Elektrodenanordnung in der expandierten Anordnung einen nicht vorragenden distalen Abschnitt hat, wobei der nicht vorragende distale Abschnitt der Elektrodenanordnung durch einen Abschnitt des Ballons gebildet ist, bei dem ein distales Ende in der expandierten Anordnung in dem Ballon invertiert ist.

2. Katheter nach Anspruch 1, wobei der inflatierbare Ballon (22) an einer distal zu dem proximalen Ende des Grats liegenden Stelle an jedem Grat (18) befestigt ist.

3. Katheter nach Anspruch 2, wobei jeder Grat (18) eine Länge hat und wobei der inflatierbare Ballon (22) entlang einem Abschnitt der Gratlänge an jedem Grat befestigt ist.

4. Katheter nach Anspruch 3, wobei der inflatierbare Ballon (22) entlang der Gratlänge an jedem Grat (18) befestigt ist.

5. Katheter nach Anspruch 1, wobei der inflatierbare Ballon (22) dazu ausgestaltet ist, eine gewünschte radiale Verteilung der Grate (18) bezüglich einander beizubehalten, wenn sie in der expandierten Anordnung sind.

6. Katheter nach Anspruch 1, wobei der inflatierbare Ballon (22) ein nachgiebiges Material, ein nicht nachgiebiges Material oder ein halbnachgiebiges Material umfasst.

7. Katheter nach Anspruch 1, ferner umfassend einen Spreizer (26) mit einem proximalen und einem distalen Ende, wobei der Spreizer verschiebbar in dem Lumen angeordnet ist, so dass die Elektrodenanordnung (16) die expandierte Anordnung hat, wenn der Spreizer in eine in Längsrichtung proximale Position bezüglich des Katheterkörpers (12) bewegt worden ist, und die zusammengeklappte Anordnung hat, wenn der Spreizer in eine in Längsrichtung distale Position bezüglich des Katheterkörpers bewegt worden ist.

8. Katheter nach Anspruch 7, wobei die Elektrodenanordnung (16) dazu ausgestaltet ist, in der expandierten Anordnung mindestens teilweise auf der Grundlage einer relativen Längsposition des Spreizers (26) bezüglich des Katheterkörpers (12) unterschiedliche Gestalten anzunehmen.

9. Katheter nach Anspruch 7, wobei der Spreizer (26) an einem distalen Ende des Ballons (22) befestigt ist.

10. Katheter nach Anspruch 1, wobei jeder Grat (18) eine vorgeformte Konfiguration hat, die der expandierten Anordnung entspricht.

11. Katheter nach Anspruch 10, wobei jeder Grat (18) ein Formgedächtnismaterial umfasst.

12. Katheter nach Anspruch 1, wobei jeder Grat (18) einen flexiblen Kern mit einer nicht leitenden Abdeckung umfasst.

13. Katheter nach Anspruch 1, wobei jeder Grat (18) eine flexible Leiterplatte umfasst.

## Revendications

1. Cathéter, comprenant :
un corps de cathéter allongé (12) présentant une extrémité proximale, une extrémité distale et une lumière ; et
un ensemble d'électrodes (16) installé à l'extrémité distale du corps de cathéter et comprenant au moins deux tiges (18) et un ballonnet gonflable (22) en communication fluidique avec la lumière de corps de cathéter allongé, chaque tige ayant au moins une électrode (20) et une extrémité proximale raccordée à l'extrémité distale du corps de cathéter, et dans lequel chaque tige présente une disposition repliée dans laquelle les tiges sont agencées généralement le long d'un axe longitudinal du corps de cathéter et une disposition déployée dans laquelle au moins une partie de chaque tige est courbée radialement vers l'extérieur à partir de l'axe longitudinal, de sorte que le ballonnet gonflable est disposé à l'intérieur des tiges et l'ensemble d'électrodes présente une portion distale non saillante lorsqu'il se trouve dans la disposition déployée, dans lequel la portion distale non saillante de l'ensemble d'électrodes est formée par une portion du ballonnet présentant une extrémité distale qui est inversée à l'intérieur du ballonnet lorsqu'il se trouve dans la disposition déployée.

2. Cathéter selon la revendication 1, dans lequel le ballonnet gonflable (22) est fixé à chaque tige (18) à un emplacement distal de l'extrémité proximale de la tige.

3. Cathéter selon la revendication 2, dans lequel chaque tige (18) présente une longueur et dans lequel le ballonnet gonflable (22) est fixé à chaque tige le long d'une portion de la longueur de tige.

4. Cathéter selon la revendication 3, dans lequel le ballonnet gonflable (22) est fixé à chaque tige (18) le long de la longueur de tige.

5. Cathéter selon la revendication 1, dans lequel le ballonnet gonflable (22) est configuré pour maintenir une distribution radiale souhaitée des tiges (18) les unes par rapport aux autres lorsqu'il se trouve dans la disposition déployée.

6. Cathéter selon la revendication 1, dans lequel le ballonnet gonflable (22) comprend un matériau élastique, un matériau non élastique ou un matériau semi-élastique.

7. Cathéter selon la revendication 1, comprenant en outre un dispositif de déploiement (26) présentant des extrémités proximale et distale, le dispositif de déploiement étant disposé de manière coulissante à l'intérieur de la lumière de sorte que l'ensemble d'électrodes (16) présente la disposition déployée lorsque le dispositif de déploiement est déplacé dans une position proximale dans le sens de la longueur par rapport au corps de cathéter (12) et présente la disposition repliée lorsque le dispositif de déploiement est déplacé dans une position distale dans le sens de la longueur par rapport au corps de cathéter.

8. Cathéter selon la revendication 7, dans lequel l'ensemble d'électrodes (16) est configuré pour adopter différentes conformations dans la disposition déployée sur la base au moins en partie d'une position longitudinale relative du dispositif de déploiement (26) par rapport au corps de cathéter (12).

9. Cathéter selon la revendication 7, dans lequel le dispositif de déploiement (26) est fixé à une extrémité distale du ballonnet (22).

10. Cathéter selon la revendication 1, dans lequel chaque tige (18) présente une configuration préformée correspondant à la disposition dilatée.

11. Cathéter selon la revendication 10, dans lequel chaque tige (18) comprend un matériau à mémoire de forme.

12. Cathéter selon la revendication 1, dans lequel chaque tige (18) comprend un noyau élastique avec un revêtement non conducteur.

13. Cathéter selon la revendication 1, dans lequel chaque tige (18) comprend un circuit souple.
